# EUROPÄISCHE PATENTANMELDUNG

(11) **EP 2 510 975 A1**
(43) Veröffentlichungstag der Anmeldung: **17.10.2012**
(21) Anmeldenummer: 12159236.4
(22) Anmeldetag: 13.03.2012
(51) Int. Cl.: A61N 1/39, A61N 1/37

(54) **Herzstimulator**

(30) Priorität: 14.04.2011 US 201161475252 P
(71) Anmelder: BIOTRONIK SE & Co. KG, 12359 Berlin (DE)
(72) Erfinder: Dörr, Thomas, 12437 Berlin (DE); Tietze, Ulrich, 13156 Berlin (DE)
(74) Vertreter: Lindner-Vogt, Karin L.

(57) **Zusammenfassung**

Die Erfindung betrifft einen implantierbaren Herzstimulator mit einer Kardioversions-/Defibrillationseinheit, die mit mindestens einer ventrikulären Wahrnehmungselektrode und einer ventrikulären Defibrillationselektrode verbunden oder zu verbinden und ausgebildet ist, Kardioversionsschocks oder Defibrillationsschocks zu generieren und abzugeben, und einer ventrikulären Wahrnehmungseinheit mit automatischer Schwellenanpassung, die mit der ventrikulären Wahrnehmungselektrode verbunden oder zu verbinden und ausgebildet ist, die Signale der Wahrnehmungselektrode zu verarbeiten und eine jeweilige Kammerkontraktion zu erfassen und im Falle einer detektierten Kammerkontraktion ein ventrikuläres Sensingsignal auszugeben. Die ventrikuläre Wahrnehmungseinheit ist ausgebildet, die Signale der Wahrnehmungselektrode mit mindestens zwei umschaltbaren Sensingschwellen wie folgt zu verarbeiten:
- nach jeder Wahrnehmung wird zunächst ein VF-Erkennungsfenster auf einer ersten unteren Schwelle als erster Sensingschwelle gestartet und
- erst nach Ablauf des VF-Erkennungsfensters ein T-Wellenausblendungsfenster auf einer oberen Schwelle als zweiter Sensingschwelle aktiviert und
- nach Ablauf des T-Wellenausblendungsfensters dann die Wahrnehmung auf einer zweiten unteren Schwelle gestartet.

## Beschreibung

Die Erfindung beschreibt eine Vorrichtung zur zuverlässigen Wahrnehmung ventrikulären Flimmerns bei gleichzeitiger effektiver Ausblendung der T-Welle zur Vermeidung von T-Wellen-Oversensing. Die Erfindung betrifft außerdem einen implantierbaren Herzstimulator für die Therapie tachykarder Herzrhythmusstörungen. Ein solcher Herzstimulator ist auch als implantierbarer Cardioverter/Defibrillator (ICD) bekannt und erfüllt in der Regel auch die Funktion eines implantierbaren Herzschrittmachers.

Tachykarde Herzrhythmusstörungen können einerseits Tachykardien im engeren Sinne sein, bei denen eine jeweils betroffene Herzkammer (Ventrikel oder Atrium) zwar geordnet, aber mit einer physiologisch unangemessen hohen Rate kontrahiert. Bei einer Fibrillation (Flimmern) kommt es dagegen zu einer ungeordneten Kontraktion der jeweiligen Herzkammer infolge kreisender Erregung, bei der die jeweils betroffene Herzkammer praktisch nicht mehr zur Förderung eines Blutvolumens beiträgt. Ventrikuläre Fibrillationen können daher tödlich sein. Sie können - wenn sie schnell und zuverlässig erkannt werden - mit einem Defibrillationsschock aus einem Defibrillator beendet werden.

Implantierbare Herzstimulatoren in Form von Herzschrittmachern oder Cardiovertern/Defibrillatoren sind grundsätzlich bekannt. Solche Herzstimulatoren sind in der Regel an Elektrodenleitungen angeschlossen, die in einer Kammer eines Herzens oder in unmittelbarer Nähe Stimulations- oder Defibrillationselektroden besitzen. Über eine Stimulationselektrode kann ein Herzschrittmacher einen elektrischen Stimulationsimpuls an das Muskelgewebe einer Herzkammer abgeben, um so eine stimulierte Kontraktion der Herzkammer hervorzurufen, sofern der Stimulationsimpuls eine ausreichende Intensität besitzt und das Herzmuskelgewebe (Myokard) sich nicht gerade in einer refraktären Phase befindet. Um auf diese Weise eine stimulierte Kontraktion einer Herzkammer auszulösen, werden üblicher Weise Elektrodenleitungen mit relativ kleinflächigen Stimulationselektroden verwendet, da es zum Auslösen einer stimulierten Kontraktion einer Herzkammer ausreicht, wenn nur ein kleiner Teil des Myokards dieser Herzkammer anfänglich stimuliert wird. Eine derartige stimulierte Kontraktion einer Herzkammer wird im Rahmen dieser Beschreibung als stimuliertes Ereignis bezeichnet. Kommt es zu einer natürlichen Kontraktion der Herzkammer, wird dies im Rahmen dieser Beschreibung als Eigenaktion oder als intrinsisches Ereignis bezeichnet. Eine Kontraktion beispielsweise des rechten Atriums eines Herzens wird als atriales Ereignis bezeichnet, welches beispielsweise ein natürliches atriales Ereignis sein kann, oder - im Falle eines atrialen Herzschrittmachers - auch ein stimuliertes atriales Ereignis. Im gleichen Sinne können natürliche (intrinsische) und stimulierte linksventrikuläre und rechtsventrikuläre Ereignisse unterschieden werden.

Eine lokale Erregung des Myokards breitet sich vom Erregungsort ausgehend per Reizleitung im Myokard aus und führt zu einer Depolarisation der Muskelzellen und damit zu einer Kontraktion des Myokards. Nach kurzer Zeit kommt es zu einer Repolarisation der Muskelzellen und damit zu einer Entspannung des Myokards. Während der Phase der Depolarisation sind die Herzmuskelzellen für eine Erregung unempfindlich, d.h. refraktär. Die mit der Depolarisation und Repolarisation einhergehenden elektrischen Potentiale können wahrgenommen und deren zeitlicher Verlauf - als Elektrokardiogram bezeichnet - ausgewertet werden. Im Elektrokardiogramm entspricht eine sogenannte R-Zacke einer Depolarisation des Ventrikel-Myokards und damit einer Kontraktion des Ventrikels. Die Repolarisation des Ventrikel-Myokards äußert sich in einer sogenannten T-Welle. Werden diese Signalmerkmale automatisch erfasst (durch eine entsprechende Wahrnehmungs- oder Sensingeinheit) geschieht dies häufig durch Schwellwertvergleich. Dabei kann es dazu kommen, dass eine T-Welle den Schwellwert zum Detektieren von R-Zacken überschreitet und somit eine vermeintliche R-Zacke detektiert wird, die tatsächlich eine T-Welle ist. Dieses Phänomen wird als T-Wellen-Oversensing bezeichnet.

Das Erfassen solcher natürlichen (intrinischen) Ereignisse erfolgt durch Ableiten der elektrischen Potenziale des Myokards der jeweiligen Herzkammer mit Hilfe von Sensingelektroden (auch als Wahrnehmungselektroden bezeichnet), die Teil einer entsprechenden Elektrodenleitung sind. Dabei können die Sensingelektroden gleichzeitig die Stimulationselektroden sein und abwechselnd als Stimulations- und als Sensingelektroden verwendet werden. Typischer Weise ist für das Sensing ein Elektrodenpaar bestehend aus einer Spitzenelektrode (Tip-Elektrode) und einer Ringelektrode vorgesehen, von denen die Spitzenelektrode auch als Stimulationselektrode dient. Auf diese Weise erfolgt eine bipolare Ableitung eines intrakardialen Elektrokardiograms (IEGM). Dabei erfolgt das Sensing und die Stimulation im Ventrikel mit Hilfe einer ventrikulären Elektrodenleitung und die Stimulation und das Sensing im Atrium (im rechten Atrium) mit einer atrialen Elektrodenleitung, die separat im jeweiligen Herzstimulator angeschlossen sind. Zusätzlich kann auch eine linksventrikuläre Elektrodenleitung vorgesehen sein, die typischer Weise über den Coronarsinus und eine von diesem abzweigenden, Lateralvene in die Nähe des linken Ventrikels ragt und dort eine kleinflächige Stimulations- und/oder Sensingelektrode aufweisen kann.

Die Sensingelektroden sind im Betrieb des Herzstimulators mit entsprechenden Sensingeinheiten verbunden, die ausgebildet sind, ein jeweiliges über eine Sensingelektrode (bzw. ein Sensingelektrodenpaar) aufgenommenes Elektrokardiogram auszuwerten und insbesondere intrisische atriale bzw. ventrikuläre Ereignisse zu detektieren, d. h. natürliche atriale oder ventrikuläre Kontraktionen. Dies geschieht beispielsweise durch Schwellwertvergleich, d. h. ein intrinsisches Ereignis wird detektiert, wenn ein jeweiliges intrakardiales Elektrokardiogram einen geeignet vorgegebenen Schwellwert überschreitet. Für die Detektion intrinsischer Ereignisse in einem implantierbaren Herzstimulator sind typischerweise Sensingeinheiten (auch als Wahrnehmungseinheiten bezeichnet) vorgesehen, die im Betrieb mit intrakardialen Elektroden verbunden sind.

Typischerweise sind im Rahmen der Wahrnehmung intrinsischer Ereignisse sogenannte Ausblendzeiten (Blankingzeiten) und Refraktärzeiten vorgesehen. Eine Ausblendzeit wird nach Erfassen einer R-Zacke gestartet, um dasselbe Ereignis nicht zweimal zu erfassen. Während der Ausblendzeit erfolgt keine Wahrnehmung von Ereignissen. Mit Erfassen einer R-Zacke wird auch eine Referaktärzeit gestartet, während der (außerhalb der Ausblendzeit) zwar intrinsische Ereignisse wahrgenommen, aber nicht als solche erfasst (detektiert) werden. Wenn die Refraktärzeit so bemessen ist, dass sie sich über den Zeitpunkt hinaus erstreckt, zu dem eine T-Welle zu erwarten ist, wird eine solche T-Welle nicht als intrinsisches Ereignis (Kontraktion der Herzkammer) erfasst.

Aus der Frequenz, mit der atriale bzw. ventrikuläre Ereignisse aufeinander folgen, kann die jeweilige intrinsische atriale Herzrate (atriale Frequenz) bzw. ventrikuläre Herzrate (Ventrikelfrequenz) abgeleitet und somit beispielsweise Tachykardien detektiert werden. T-Wellen-Oversensing stellt hier ein Problem dar, da T-Wellen-Oversensing das vermeintliche Vorhandensein einer Tachykardie suggerieren kann.

Der Erfindung liegt die Aufgabe zugrunde, die Erkennung von ventrikulärem Flimmern bei gleichzeitig guter Unterdrückung von T-Wellen-Oversensing zu verbessern. Die Lösung bietet insbesondere bei VF-Signalen mit stark wechselnden Amplituden einen Vorteil.

Eingangsstufen moderner implantierbarer Cardioverter/Defibrillatoren (ICDs) bzw. Herzstimulatoren für die kardiale Resynchronisationstherapie (CRT-Ds) bieten zwar bereits Algorithmen zur automatischen Anpassung der Wahrnehmungsschwellen an die Signalgegebenheiten. Diese arbeiten typischerweise mit Wahrnehmungsschwellen, die von den gemessenen Signalamplituden abgeleitet werden. Ferner wird für die Ausblendung der T-Welle nach einer R-Wellenerkennung häufig mit einer sogenannten oberen Schwelle (z. B. 75% der R-Wellenamplitude) gearbeitet, die nach der erwarteten Zeit der T-Welle (z. B. nach 360ms) dann auf eine untere Schwelle (z.B. 25%) umgeschaltet wird. Damit wird erreicht, dass die T-Welle ausgeblendet werden kann, indem diese unterschwellig bleibt und trotzdem mit der danach aktivierten unteren Schwelle feinere Flimmerwellen nachfolgend erfasst werden können.

Die bekannten Verfahren haben jedoch den Nachteil, dass durch die Verwendung einer sog. Oberen Schwelle von beispielsweise 75% für eine Zeit von 360ms Flimmersignale mit stark wechselnden Amplituden nur unzureichend wahrgenommen werden können. Wird die obere Schwelle jedoch kleiner eingestellt (z. B. 50%), kann wiederum eine große T-Welle nicht ausgeblendet werden, so dass bei Patienten mit großen T-Wellen eine eingeschränkte VF-Wahrnehmung als Restrisiko bleibt.

Erfindungsgemäß wird die Aufgabe, eine effektive T-Wellenunterdrückung mit einer sehr guten VF-Wahrnehmung zu kombinieren einen implantierbaren Herzstimulator, beispielsweise einen Kardioverter/Defibrillator (ICD, CRT-D), gelöst, der eine Wahrnehmungseinheit mit automatischer Schwellenanpassung aufweist, die mit mindestens einer ventrikulären Wahrnehmungselektrode und einer ventrikulären Defibrillationselektrode verbunden oder zu verbinden ist, wobei über die Wahrnehmungselektrode aufgenommene Signale in der Wahrnehmungseinheit mit automatischer Schwellenanpassung verarbeitet werden und diese Wahrnehmungseinheit mit mindestens 2 umschaltbaren Sensingschwellen wie folgt arbeitet:
- nach jeder Wahrnehmung wird zunächst ein erfindungsgemäßes VF-Erkennungsfenster (VF-Erkennungsfenster) auf einer ersten unteren Schwelle gestartet und
- erst nach Ablauf des VF-Erkennungsfensters (d. h. wenn im VF-Erkennungsfenster keine Überschreitung der ersten Sensingschwelle erfolgte) wird ein T-Wellenausblendungsfenster auf einer oberen Schwelle als zweiter Sensingschwelle aktiviert und
- nach Ablauf des T-Wellenausblendungsfensters dann die Wahrnehmung auf einer zweiten unteren Schwelle gestartet.

Hierbei können die erste und die zweite untere Schwelle identisch und von der zweiten Sensingschwelle verschieden sein oder die erste und die zweite untere Schwelle sind voneinander und von der zweiten Sensingschwelle verschieden. Die Schwellen und Sensingschwellen werden nachfolgend auch als Wahrnehmungsschwellen bezeichnet und stellen jeweils untere Schwellwerte dar, deren Überschreiten ein Ereignis auslösen, sofern dies außerhalb einer Blankingzeit erfolgt.

Der implantierbare Herzstimulator ist vorzugsweise ein Einkammer-, Zweikammer-, Dreikammer-ICD.

Vorzugsweise weist der Herzstimulator eine Steuereinheit auf, die ausgebildet ist, eine jeweilige Dauer des VF-Erkennungsfensters abhängig von einer jeweils gemessenen Herzfrequenz einzustellen, sodass sich das VF-Erkennungsfenster bei höherer Herzrate verkürzt und bei einer vergleichsweise niedrigeren Herzrate verlängert. Beispielsweise kann das VF-Erkennungsfenster jeweils eine Dauer haben, die einem festgelegten Bruchteil einer über eine vorgegebene Anzahl der vorangegangen Herzzyklen gemittelte Herzyklusdauer (Kehrwert der Herzfrequenz) entspricht. Auf diese Weise kann die Dauer des VF-Erkennungsfensters an die jeweiligen konkreten physiologischen Gegebenheiten angepasst werden.

Vorzugsweise erfolgt eine Anpassung der Dauer des VF-Erkennungsfensters nur dann abhängig von einer jeweils gemessenen Herzfrequenz, wenn in einem jeweils vorangegangenen Herzzyklus eine T-Wellenausblendung aktiviert wurde (d. h. eine T-Wellen-Ausblendfenster gestartet wurde), also innerhalb eines jeweils vorangegangenen Herzzyklus keine Schwellwertüberschreitung im VF Erkennungsfenster erfolgt ist.

Vorzugsweise ist die Wahrnehmungseinheit derart mit einer Wahrnehmungselektrode verbunden oder zu verbinden, dass eine Erfassung intrakardialer elektrischer Potentiale bipolar - das heißt, über zwei relativ kleinflächige und benachbarte Elektrodenpole (Ring- oder Tip-Elektroden) einer entsprechenden Wahrnehmungselektrode - erfolgt.

Alternativ kann die Wahrnehmungseinheit auch derart mit einer Wahrnehmungselektrode verbunden oder zu verbinden sein, dass eine Erfassung intrakardialer elektrischer Potentiale unipolar - d. h., über einen einzigen relativ kleinflächigen Elektrodenpol (Ring- oder Tip-Elektrode) einer entsprechenden Wahrnehmungselektrode und einen vergleichsweise größeren Neutralpol (beispielsweise von einem Gehäuse des Herzstimulators gebildet) - erfolgt.

Alternativ oder zusätzlich kann die Wahrnehmungseinheit auch derart mit einer Wahrnehmungselektrode verbunden oder zu verbinden sein, dass sie ein Fernfeld intrakardialer elektrischer Potentiale erfasst. Dies geschieht beispielsweise über zwei relativ großfläche Elektrodenpole, z. B. einer Defibrillationswendel an der Wahrnehmungselektrode und dem Gehäuse des Herzstimulators als weiterer Elektrode.

Schließlich kann die Wahrnehmungseinheit auch derart mit mehreren Wahrnehmungselektroden verbunden oder zu verbinden sein, dass eine Erfassung intrakardialer elektrischer Potentiale über mehrere Vektoren erfolgt, d. h. die Erfassung der intrakardialen Potentiale erfolgt über mehr als zwei Elektrodenpole, wobei das zwischen jeweils zwei Elektrodenpolen herrschende Potential jeweils einen Vektor bildet.

Die Erfindung soll nun anhand eines Ausführungsbeispiels mit Bezug auf die Abbildungen näher erläutert werden.

Von den Figuren zeigt.
- FIG.1:: einen Zweikammer-Herzschrittmacher und implantierbaren Kardioverter/Defibrillator (ICD) als implantierbaren Herzstimulator;
- FIG. 2:: einige Bestandteile des Herzstimulators in Form eines vereinfachten Blockdiagramms;
- FIG. 3:: einen Dreikammer-Herzschrittmacher und implantierbaren Kardioverter/Defibrillator (ICD) als implantierbaren Herzstimulator;
- FIG. 4:: ein Ausschnitt eines Blockschaltbilds eines Zweikammer-ICD, Blockschaltbild des ICD-Systems mit VF-Erkennungsfenster;
- FIG. 5:: ein typisches Beispiel eines T-Wellen-Oversensings;
- FIG. 6:: ein Beispiel für eine T-Wellenunterdrückung ohne VF-Erkennungsfenster;
- FIG. 7:: ein Beispiel für ein VF-Undersensing ohne VF-Erkennungsfenster ;
- FIG. 8:: ein Beispiel für eine VF-Erkennung ohne VF-Erkennungsfenster;
- FIG. 9 :: ein Beispiel für eine T-Wellenunterdrückung mit VF-Erkennungsfenster;
- FIG. 10:: ein Beispiel für eine VF-Erkennung mit VF-Erkennungsfenster;
- FIG. 11:: ein Beispiel für eine VT-Erkennung mit VF-Erkennungsfenster; und
- FIG. 12:: ein typisches Timing mit Blanking und Refraktärzeit;

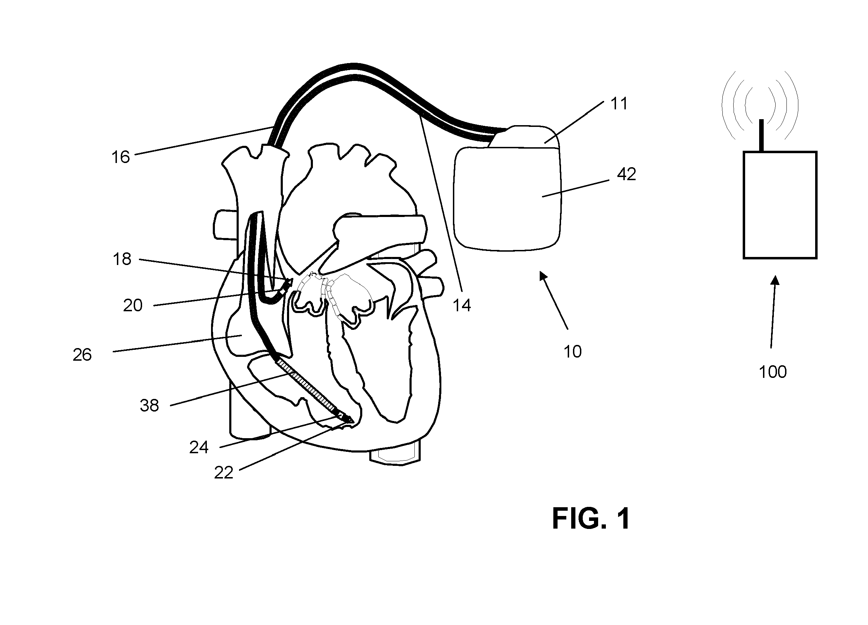

Abbildung 1 zeigt einen Zweikammer-Herzschrittmacher und implantierbaren Kardioverter/Defibrillator (ICD) als implantierbaren Herzstimulator 10. Dieser Zweikammer-Herzschrittmacher ist über Elektrodenleitungen 14 und 16 mit Stimulations- und Sensingelektroden 18 und 20 bzw. 22 und 24 im Atrium bzw. Ventrikel eines Herzens verbunden und kann auf diese Weise Stimulationsimpulse an das Herz abgeben und elektrische Potentiale vom Herzen aufnehmen.

Außerdem ist ein externes Gerät 100 in der Nähe des implantierbaren Herzstimulators 10 dargestellt.

Die Elektrodenleitungen 14 und 16 sind über bekannte, standardisierte Steckverbindungen mit Kontaktbuchsen in einem Header (Anschlussgehäuse) 11 des Herzstimulators 10 elektrisch verbunden. Auf diese Weise sind die Elektrodenleitungen 14 und 16 auch mit elektronischen Komponenten im Inneren eines hermetisch dichten Metallgehäuses 42 des Herzstimulators 10 verbunden. Diese Komponenten sind nachfolgend detaillierter schematisch dargestellt und bestimmen die erfindungsgemäße Funktionsweise des Herzstimulators 10.

Die Elektrodenleitung 14 ist eine rechtsatriale Elektrodenleitung und besitzt an ihrem distalen Ende eine atriale Spitzenelektrode RA Tip 18 sowie in kurzer Entfernung davon eine atriale Ringelektrode RA Ring 20, die beide im rechten Atrium 26 des Herzens 12 platziert sind.

Die Elektrodenleitung 16 ist eine rechtsventrikuläre Elektrodenleitung und besitzt an ihrem distalen Ende eine rechtsventrikuläre Spitzenelektrode RV Tip 22 und in unmittelbarer Nähe eine rechtsventrikuläre Ringelektrode RV Ring 24. Beide Elektroden sind im Apex des rechten Ventrikels des Herzens angeordnet.

Außerdem trägt die rechtsventrikuläre Elektrodenleitung 16 noch eine rechtsventrikuläre Schockwendel RV Schock 38 als großflächige Elektrode zur Abgabe von Defibrillationsschocks.

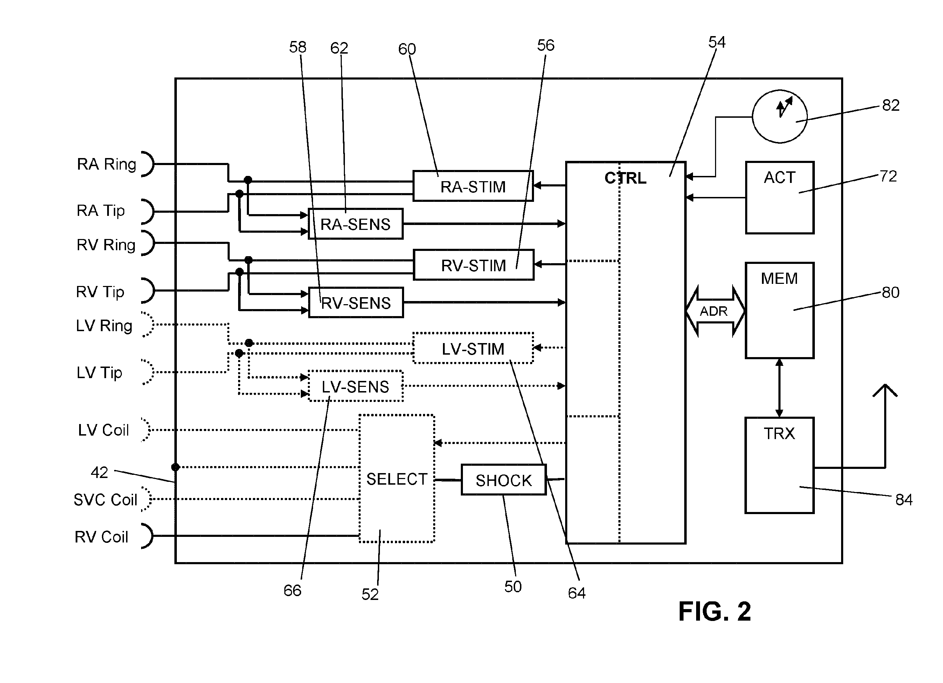

In Abbildung 2 sind einige der wesentlichen Funktionseinheiten des Herzstimulators 10 dargestellt. In punktierter Liniendarstellung sind außerdem weitere Komponenten dargestellt, wie sie bei einer alternativen Ausführungsvariante eines implantierbaren Herzstimulators zusätzlich vorgesehen sein können.

Auf der linken Seite sind die elektrischen Anschlüsse für die verschiedenen Elektroden 18, 20, 22, 24, und 38 dargestellt. Die Schockelektrode (Schockwendel) 38 ist mit einem Schockimpulsgenerator 50 verbunden. Der Schockimpulsgenerator 50 ist mit einer Stimulationssteuereinheit 54 verbunden, die den Schockimpulsgenerator 50 bei Bedarf zur Erzeugung und Abgabe eines Kardioversions- oder Defibrillationsschocks ansteuert.

Der Anschluss für die rechtsventrikuläre Tippelektrode RV Tip 18 sowie der Anschluss für die rechtsventrikuläre Ringelektrode RV Ring 20 sind jeweils sowohl mit einer rechtsventrikulären Stimulationseinheit 56 als auch mit einer rechtsventrikulären Sensingeinheit 58 verbunden. Sowohl die rechtsventrikuläre Stimulationseinheit 56 als auch die rechtsventrikuläre Sensingeinheit 58 sind jeweils mit der Stimulationssteuereinheit 54 verbunden.

Die rechtsventrikuläre Stimulationseinheit 56 ist dazu ausgebildet, auf ein Ansteuersignal der Stimulationssteuereinheit 54 hin einen rechtsventrikulären Stimulationsimpuls zu erzeugen und im Anschluss über die rechtsventrikuläre Ringelektrode RV Ring 20 und die rechtsventrikuläre Tippelektrode RV Tip 18 abzugeben. Alternativ ist es auch möglich, dass das Gehäuse 42 des Herzstimulators 10 eine neutrale Elektrode bildet und die rechtsventrikuläre Stimulationseinheit 56 mit dem Anschluss für die rechtsventrikuläre Ringelektrode RV Tip 18 und dem Gehäuse 42 als andere Elektrode zur Abgabe eines Stimulationsimpulses verbunden ist. Ein rechtsventrikulärer Stimulationsimpuls unterscheidet sich von einem Defibrillationsschock dadurch, dass der Stimulationsimpuls eine wesentlich geringere Impulsstärke besitzt, so dass er nicht wie ein Defibrillationsschock auf einen Schlag das vollständige Herzgewebe (Myokard) einer Herzkammer erregt, sondern nur die Herzmuskelzellen in unmittelbarer Umgebung der rechtsventrikulären Spitzenelektrode RV Tip 18. Diese Erregung breitet sich dann durch natürliche Reizleitung über den gesamten Ventrikel weiter aus und sorgt so für eine stimulierte Kontraktion des Ventrikels.

Die rechtsventrikuläre Sensingeinheit 58 ist dazu ausgebildet, an dem Anschluss für die rechtsventrikuläre Ringelektrode RV Ring 20 und die rechtsventrikuläre Tippelektrode RV Tip 18 anliegenden elektrischen Potentiale zunächst durch einen Eingangsverstärker zu verstärken und zu filtern. Weiterhin ist die rechtsventrikuläre Sensingeinheit ausgebildet, den Verlauf der an ihren Eingängen anliegenden elektrischen Signale derart auszuwerten, dass die rechtsventrikuläre Sensingeinheit 58 selbsttätig eine natürliche (intrinsische), d. h. selbsttätige Kontraktion des rechten Ventrikels detektiert. Dies kann beispielsweise dadurch geschehen, dass der Verlauf des an den Eingängen der rechtsventrikulären Sensingeinheit 58 anliegenden Signals mit einem Schwellwert verglichen wird. Typischerweise ist die größte Amplitude des Signals in Form der sogenannten R Zacke kennzeichnend für eine natürliche Kontraktion des rechten Ventrikels, die durch Schwellwertvergleich detektiert werden kann. Die rechtsventrikuläre Sensingeinheit 58 gibt daraufhin ein entsprechendes, eine natürliche Kontraktion des rechten Ventrikels anzeigendes Ausgangssignal an die Stimulationssteuereinheit 54 aus.

In analoger Weise sind der Anschluss für die rechtsatriale Tippelektrode RA Tip22 und der Anschluss für die rechtsatriale Ringelektrode RA Ring 24 sowohl mit einer rechtsatrialen Stimulationseinheit 60 als auch mit einer rechtsatrialen Sensingeinheit 62 verbunden, die jeweils ihrerseits wiederum mit der Stimulationssteuereinheit 54 verbunden sind. Die rechtsatriale Stimulationseinheit 60 ist dazu ausgebildet, Stimulationsimpulse zu erzeugen, deren Stärke ausreicht, das rechtsatriale Myokard zu erregen. Die rechtsatrialen Stimulationsimpulse können dabei eine andere Impulsstärke besitzen als die rechtsventrikulären Stimulationsimpulse. Die rechtsatriale Sensingeinheit 62 ist ausgebildet, aus dem Verlauf des an ihren Eingängen anliegenden Differenzsignals eine sogenannte P-Welle zu detektieren, die eine natürliche (intrinsische) Kontraktion des rechten Atriums kennzeichnet. Detektiert die rechtsatriale Sensingeinheit 62 eine entsprechende P-Welle, erzeugt sie ein Ausgangssignal und gibt dieses an die Stimulationssteuereinheit 54 weiter, welches eine natürliche Kontraktion des rechten Atriums kennzeichnet.

Als weiterer Bestandteil des Herzstimulators 10 ist ein Aktivitätssensor 72 mit der Stimulationssteuereinheit 54 verbunden. Der Aktivitätssensor 72 ist dazu ausgebildet, ein von der körperlichen Aktivität eines Patienten abhängiges Signal, zum Beispiel ein Bewegungssignal, zu erfassen und ein entsprechendes, die körperliche Aktivität des Patienten anzeigendes Signal an die Stimulationssteuereinheit 54 auszugeben. Dies erlaubt es, dass die Stimulationssteuereinheit 54 das Timing der Stimulationsimpulse an den Bedarf des Patienten (hämodynamischer Bedarf) anpasst.

Weiterhin umfasst der Herzstimulator 10 eine Speichereinheit 80, die mit der Stimulationssteuereinheit 54 verbunden ist und es erlaubt, von der Stimulationssteuereinheit 54 erzeugte oder ausgewertete Signale zu speichern. Andererseits erlaubt es die Speichereinheit 80, Steuerprogramme für die Stimulationssteuereinheit 54 in veränderbarer Form zu speichern. Des Weiteren ist die Stimulationssteuereinheit 54 mit einem Zeitgeber 82 verbunden.

Der Herzstimulator 10 verfügt über mindestens eine bidirektionale Telemetrieschnittstelle 84, um gespeicherte Daten vom elektronischen Implantat an ein externes Gerät 100 übertragen zu können und umgekehrt auch Programmiereinstellungen und Therapiekommandos von diesem externen Gerät 100 empfangen zu können.

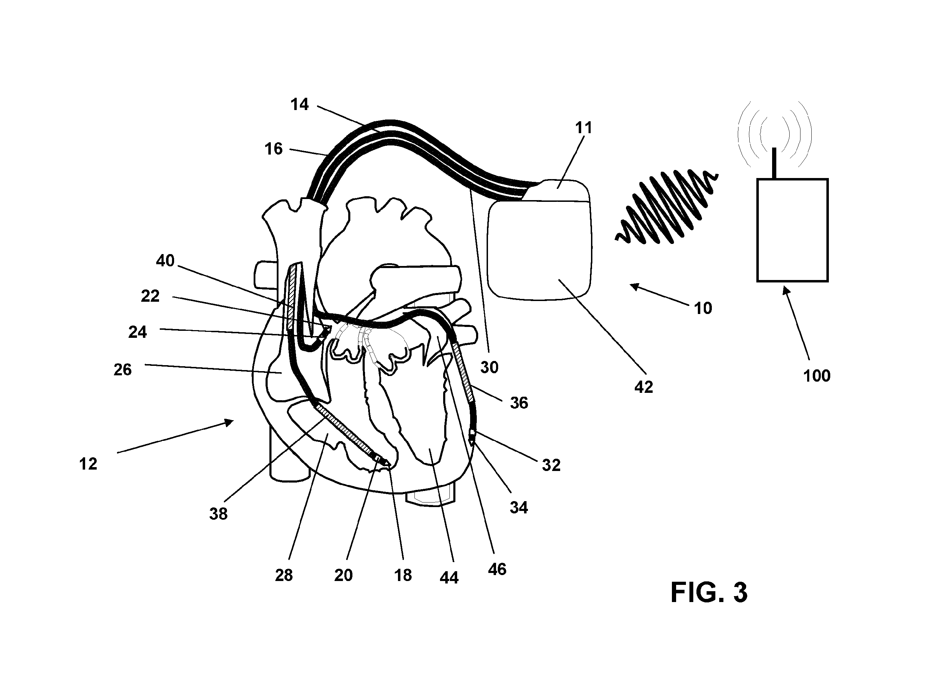

In Abbildung 3 ist eine alternative Ausführungsvariante eines implantierbaren Herzstimulators in Form eines bi-ventrikulären Dreikammer-Herzstimulators / ICDs 10 dargestellt. Dieser ist über seinen Anschlussblock 11 (Header) mit einer rechtsventrikulären 16, einer rechtsatrialen 14 und zusätzlich einer linksventrikulären Elektrodenleitung 30 verbunden.

Diese Elektrodenleitungen werden im Herzen 12 dauerhaft implantiert. Die rechtsventrikuläre Elektrodenleitung 16 weist dabei am distalen Ende eine bipolare Stimulations- und Wahrnehmungselektrode mit Tip- 18 und Ringelektrode 20 auf. Ferner ist diese Elektrodenleitung mit einer distalen Schockwendel 38 und zusätzlich einer proximalen Schockwendel 40 ausgestattet. Die distale Schockwendel 38 ist dabei so angeordnet, dass diese im rechten Ventrikel 28 liegt. Die proximale Schockwendel 40 liegt im oberen Teil des rechten Atriums 26. Die atriale Elektrodenleitung 14 weist am distalen Ende eine bipolare Stimulations- und Wahrnehmungselektrode, bestehen aus Tip- 22 und Ringelektrode 24 auf, implantiert im rechten Atrium 26.

Die Elektrodenleitung 30 ist eine linksventrikuläre Elektrodenleitung an deren distalem Ende eine linksventrikuläre Spitzenelektrode LV Tip 34 sowie in deren Nähe eine linksventrikuläre Ringelektrode LV Ring 32 angeordnet ist. Außerdem trägt die linksventrikuläre Elektrodenleitung 30 eine linksventrikuläre Schockwendel 36 zur Abgabe von Defibrillationsschocks an den linken Ventrikel. Diese Schockwendel 36 ist dabei so angeordnet, dass diese vom linken Ventrikel 44 bis hinauf zum linken Vorhof 46 reicht. Eine weitere Elektrode für die Schockabgabe stellt das elektrisch aktive Gehäuse 42 des Implantates 10 dar.

Die linksventrikuläre Elektrodenleitung 30 wird vom rechten Atrium 26 des Herzens 12 aus über den Koronarsinus in eine von diesem abzweigende Lateralvene geführt und wird deswegen auch als Koronarsinuselektrodenleitung oder CS-Elektrodenleitung bezeichnet.

Wie aus Abbildung 2 anhand der punktiert dargestellten Komponenten zu entnehmen ist, sind auch der Anschluss für die linksventrikuläre Tippelektrode LV Tip 34 und der Anschluss für die linksventrikuläre Ringelektrode LV Ring 32 mit einer linksventrikulären Stimulationseinheit 64 und einer linksventrikulären Sensingeinheit 66 verbunden. Die linksventrikuläre Stimulationseinheit 64 und die linksventrikuläre Sensingeinheit 66 sind ebenso mit der Stimulationssteuereinheit 54 verbunden. Beide funktionieren ähnlich wie die bereits beschriebenen Stimulationseinheiten 56 und 60 und Sensingeinheiten 58 und 62.

Die linksventrikuläre Schockwendel 36 ist über einen Anschluss LV-COII, und eine Elektrodenauswahleinheit 52 ebenfalls mit dem Schockgenerator 50 verbunden. Mittels der E-lektrodenauswahleinheit 52 kann die Steuereinheit 54 zwei oder mehr Elektroden (einschließlich des leitenden Gehäuses 42) auswählen, über die ein Schock abgegeben werden soll.

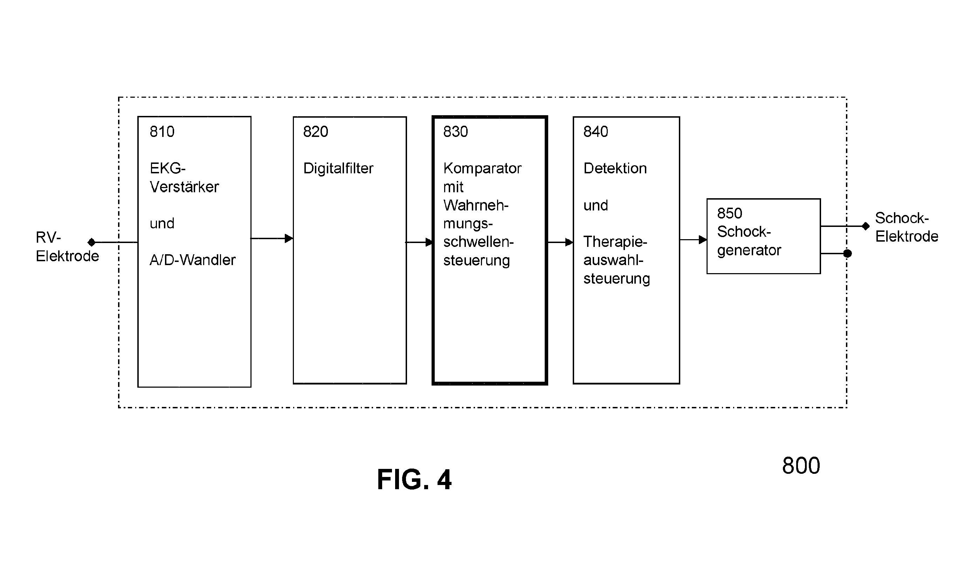

In Abbildung 4 ist als ein Ausschnitt eines detaillierteren Blockschaltbilds eines Zweikammer-ICD, ein Blockschaltbild des ICD-Systems mit VF-Erkennungsfenster dargestellt. Die rechtsventrikuläre Elektrode (RV) ist mit einer EKG-Verstärker und Analog/Digital-Wandlereinheit verbunden (810). Das digitalisierte IEGM-Signal wird anschließend gefiltert (820) und dann der erfindungsgemäßen Komparatoreinheit (830) zugeführt. Diese Einheit ermittelt dann die Wahrnehmungsereignisse unter Anwendung der beschriebenen Schwellenanpassung inklusive VF-Erkennungsfenster. Die Wahrnehmungssignale werden dann in einer Detektions- und Therapiesteuereinheit (840) ausgewertet und bei Bedarf eine antitachykarde Therapie, wie z.B. eine Defibrillation (850) abgegeben.

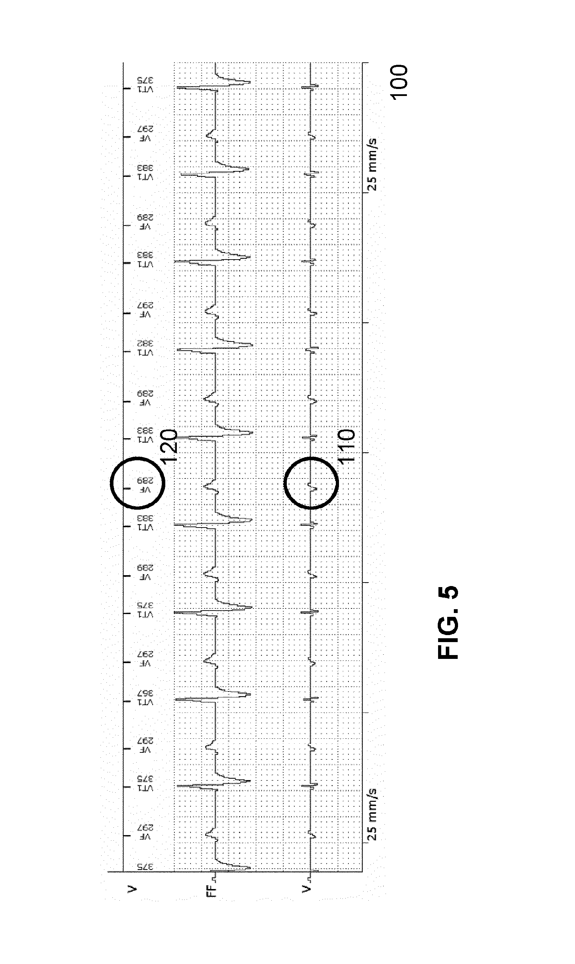

In der Abbildung 5 ist ein typisches Beispiel eines T-Wellen-Oversensings dargestellt. Im aufgezeichneten EKG und Markerstreifen (100) sind in der ventrikulären IEGM-Morphologie ausgeprägte T-Wellen (110) zu sehen, deren Amplituden größer als 50% der vorangegangenen R-Wellenamplituden betragen. In der Standardeinstellung führt dies dann zu inadäquater Wahrnehmung der T-Wellen (120) und einer damit verbundenen Rhythmusfehlbewertung als ventrikuläres Flimmern (VF). Folge dieses Oversensings können dann inadäquate Schockabgaben des ICD sein.

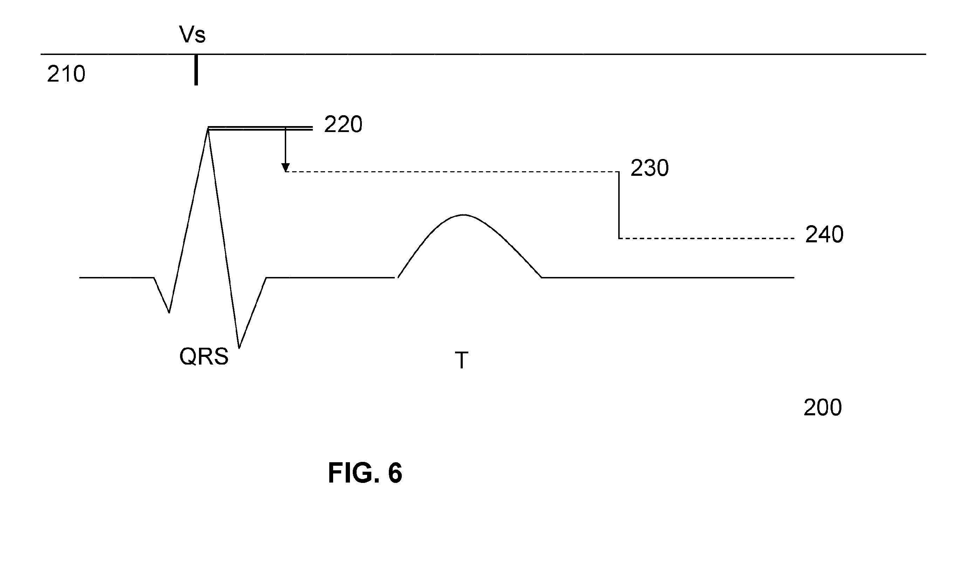

In der Abbildung 6 ist der Stand der Technik dargestellt. Um das in Abbildung 5 gezeigte T-Wellen-Oversensing zu vermeiden, bietet der Stand der Technik die Möglichkeit, die Wahrnehmungsschwellen für ein ICD-System 2-stufig umzuschalten. Hier wird nach jeder Wahrnehmung Vs (im Markerkanal 210) der Maximalwert der IEGM-Signalamplitude 220 bestimmt und davon wird zunächst eine sogenannte obere Wahrnehmungsschwelle 230 abgeleitet und aktiviert. Diese Schwelle kann z.B. in Prozent des Maximalwertes eingestellt werden und müsste zur Vermeidung des in Abbildung 1 gezeigten T-Wellen-Oversensings z.B. 75% betragen und so lange aktiviert sein, bis die T-Welle abgeklungen ist (z.B. 360ms). Anschließend wird dann eine sogenannte untere Schwelle 240 aktiviert, die typischerweise 25% des Maximalwertes 220 beträgt. Diese Schwelle gestattet die Wahrnehmung von ventrikulären Flimmersignalen mit deutlich kleinerer Signalamplitude als die reguläre R-Welle (QRS).

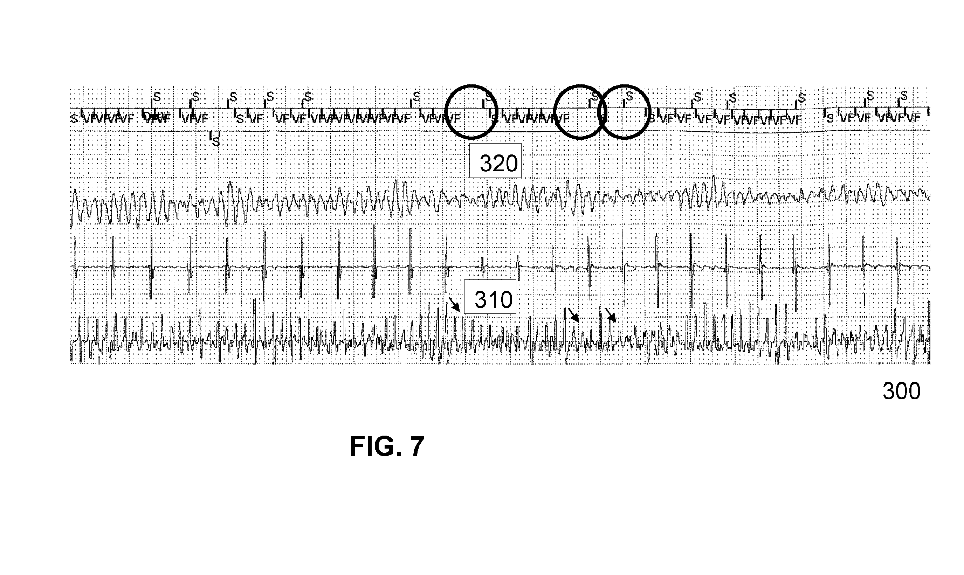

In der Abbildung 7 ist der Nachteil der erhöhten T-Wellenunterdrückung mit einer oberen Schwelle von 75% dargestellt. Die abgebildete IEGM-Aufzeichnung 300 zeigt im ventrikulären Kanal deutlich schwankende VF-Signalamplituden (siehe Pfeile nahe der Bezugsziffer 310), wobei die Amplituden um mehr als 25% schwanken. Diese führt aufgrund der erhöhten oberen Schwelle häufig zu Sensingausfällen (Undersensing) im ventrikulären Markerkanal (oberes Diagramm 320) und damit zu einer deutlich verzögerten Detektion des ventrikulären Flimmerns.

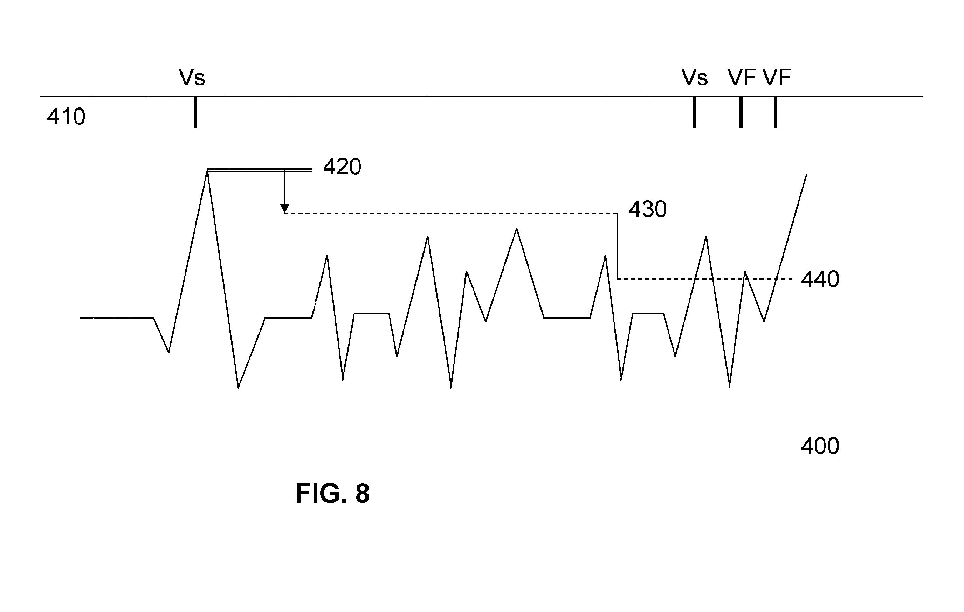

In der Abbildung 8 ist nochmals der Mechanismus des VF-Untersensings durch eine zu hohe obere Schwelle 430 von 75% des IEGM-Maximalwertes 420 dargestellt. Das nach einer Wahrnehmung mit relativ großer Amplitude einsetzende ventrikuläre Flimmern mit wechselnder Amplitude wird zunächst nicht erkannt, da alle Signale unterhalb einer oberen Schwelle 430 liegen. Erst nach Umschalten auf eine untere Schwelle 440 werden die Flimmersignale wahrgenommen und im Markerkanal (oberes Diagramm 410) entsprechend dargestellt (VF). Allerdings wird der erste Marker aufgrund des zu großen Abstandes zum vorhergehenden Marker als Vs annotiert und so die VF-Erkennung verzögert. Bei häufig wechselnden Signalamplituden während des Flimmerns kann sich die Situation mehrfach wiederholen.

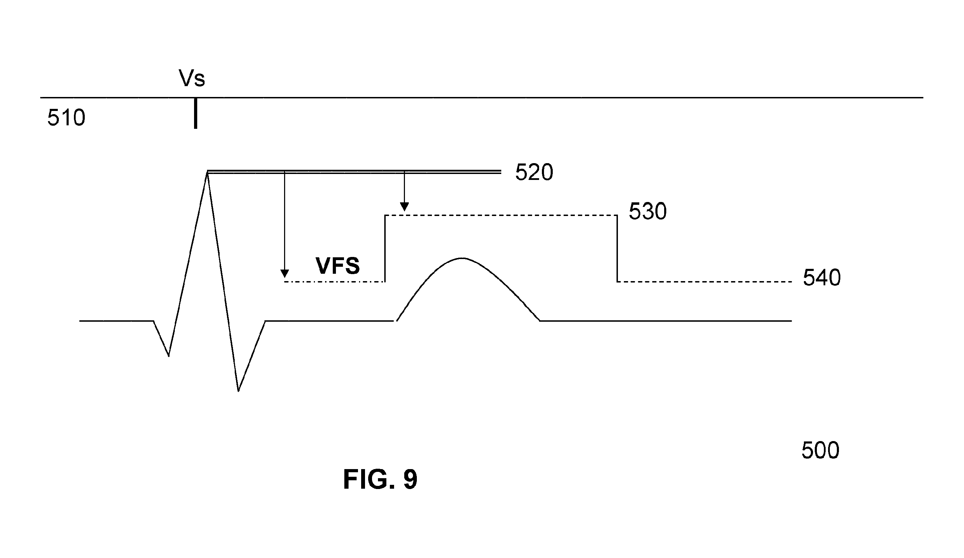

In der Abbildung 9 ist nun das erfindungsgemäße zusätzliche VF-Erkennungsfenster (VF-Erkennungsfenster) dargestellt. Dieses wird zunächst immer nach einer ventrikulären Wahrnehmung (z. B. Vs) gestartet. Innerhalb des VF-Erkennungsfensters ist entweder die untere Schwelle 540 oder eine gegenüber der oberen Schwelle 530 deutlich kleinere Schwelle wirksam. Die Ausblendung der T-Welle erfolgt wie bisher über die obere Schwelle 530, die im Anschluss an das VF-Erkennungsfenster gestartet wird. Die Dauer des VF-Erkennungsfensters ist dabei immer so bemessen, dass innerhalb des VF-Erkennungsfensters noch keine T-Welle erwartet werden kann. D. h. das VF-Erkennungsfenster endet zwischen 150 ms und 250 ms nach einer Wahrnehmung. Optional kann die Dauer des VF-Erkennungsfensters von der Herzfrequenz abhängig sein und bei höheren Herzraten verkürzt werden. Am Ende der T-Wellenausblendzeit wird wie bisher dann die untere Schwelle 540 aktiviert.

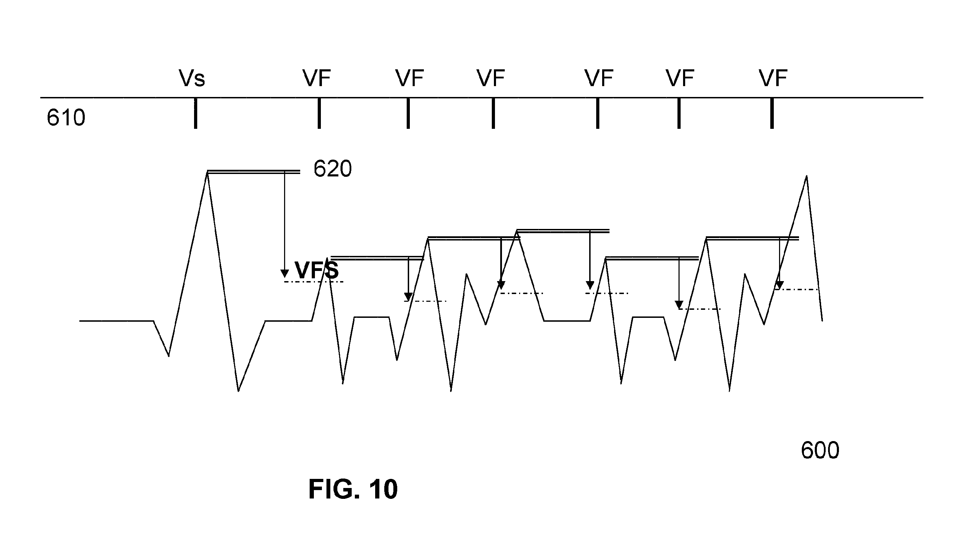

In Abbildung 10 ist die Wirksamkeit des VF-Erkennungsfensters bei einer ventrikulären Fibrillation (VF) dargestellt. Die Flimmersignale werden trotz stark schwankender Amplituden zuverlässig im Markerkanal 610 als VF annotiert, da mit jeder Wahrnehmung das VF-Erkennungsfenster erneut gestartet wird und damit die obere Schwelle nicht wirksam wird.

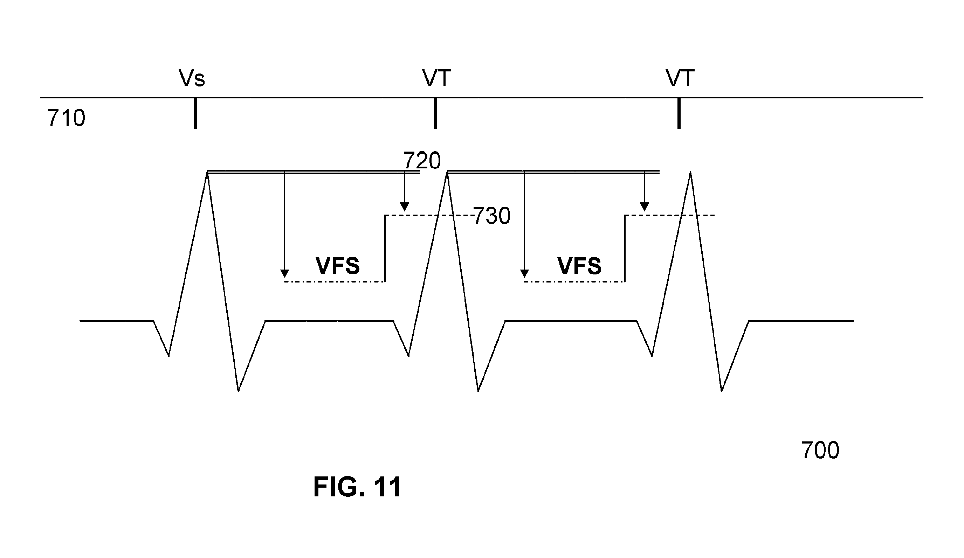

In Abbildung 11 ist dargestellt, dass die Wahrnehmung einer ventrikulären Tachykardie (VT) bei Anwendung eines VF-Erkennungsfensters unverändert erfolgt. Bei einer VT kann davon ausgegangen werden, dass die Signalamplituden weit weniger schwanken als bei einer ventrikulären Fibrillation VF, sodass hier die von der Maximalamplitude 720 abgeleitete obere Schwelle 730 als Wahrnehmungskriterium anwendbar ist.

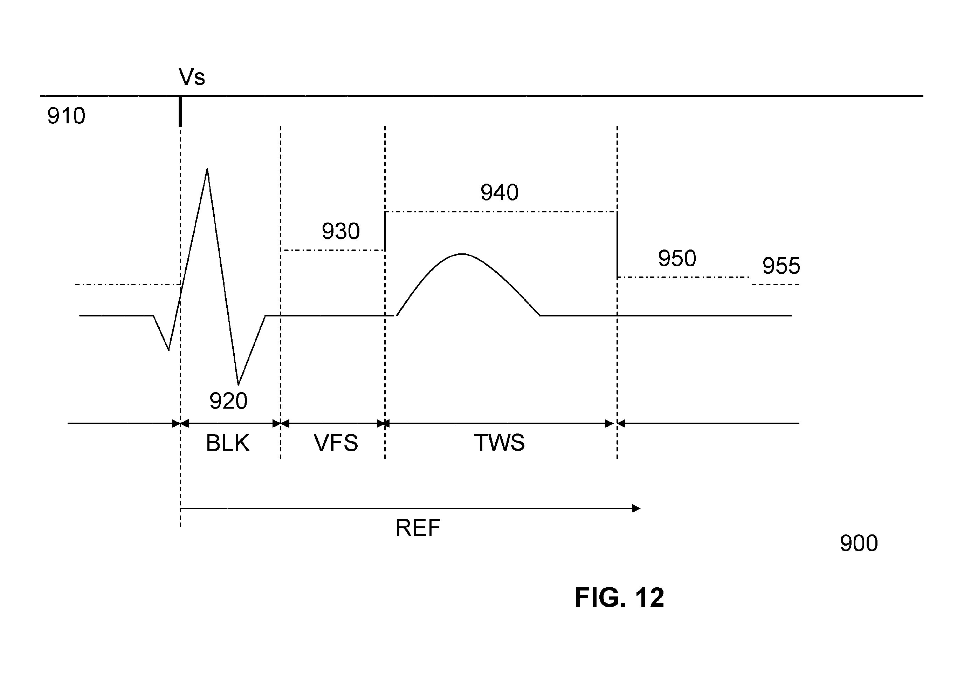

In der Abbildung 12 ist die ventrikuläre Wahrnehmungsschwellensteuerung einschließlich der Ausblendzeit (Blanking: BLK) und Refraktärzeit (REF) dargestellt.

Nach Überschreitung der Wahrnehmungsschwelle wird im Markerkanal 910 zunächst ein ventrikulärer Sense Vs ausgelöst. Mit diesem Vs startet dann eine ventrikuläre Ausblendzeit BLK 920, um eine mehrfache Wahrnehmung des QRS-Komplexes zu vermeiden. Während dieser Zeit kann keine, den Zeitgeber oder die Tachyarrhythmieklassifikation beeinflussende (also startende oder zurücksetzende) Wahrnehmung ausgelöst werden, d.h. es ist kein weiterer Vs während BLK möglich. Nach Ablauf dieser Ausblendzeit startet die Wahrnehmung mit der erfindungsgemäßen VF-Erkennungsschwelle VFS 930, um ein ggf. aufgetretenes Ventrikelflimmern mit kleinen Amplituden wahrnehmen zu können. Ist dies nicht vorhanden, wird nach Ablauf der einstellbaren VF-Erkennungsfenster-Zeit die Wahrnehmungsschwelle auf den T-Wellen-Ausblendwert TWS 940 für die programmierte T-Wellen-Ausblendzeit angehoben und danach auf die sogenannte untere Wahrnehmungsschwelle 950 reduziert. Diese untere Wahrnehmungsschwelle kann üblicherweise nach definierten Zeitintervallen weiter reduziert werden (siehe Bezugsziffer 955).

Alle Wahrnehmungsschwellen können wahlweise feste oder von der QRS-Signal-Maximalamplitude abhängige Werte einnehmen.

Die dargestellte Refraktärzeit (REF) wird mit dem ventrikulären Sense (Vs) gestartet. Ein Vs innerhalb der Refraktärzeit wird zwar für die tachykarde Rhythmusbewertung ausgewertet, führt jedoch nicht zur Beeinflussung des Schrittmacherzeitgebers. Die Refraktärzeit (REF) ist unabhängig von der Steuerung der ventrikulären Wahrnehmungsschwellen.

Die hier beschriebene Lösung verbessert die Wahrnehmung ventrikulären Flimmerns bei gleichzeitiger hoher T-Wellenunterdrückung sehr deutlich. Damit können zukünftige ICD-Systeme realisiert werden, die keine manuelle Anpassung der Wahrnehmungsparameter mehr erfordern (automatisches Implantat). Ebenso ist diese Lösung für die Verwendung unipolarer ICD-Sensingelektroden eine Voraussetzung, da hier die Ausprägung der T-Welle und vor allem die VF-Signalschwankungen als besonders kritisch einzuschätzen sind. Ebenso kann das Verfahren bei subkutan implantierten ICD-Systemen angewendet werden.

## Patentansprüche

1. Implantierbarer Herzstimulator mit:
- einer Kardioversions-/Defibrillationseinheit, die mit mindestens einer ventrikulären Wahrnehmungselektrode und einer ventrikulären Defibrillationselektrode verbunden oder zu verbinden und ausgebildet ist, Kardioversionsschocks oder Defibrillationsschocks zu generieren und abzugeben,
- einer ventrikulären Wahrnehmungseinheit mit automatischer Schwellenanpassung, die mit der ventrikulären Wahrnehmungselektrode verbunden oder zu verbinden und ausgebildet ist, die Signale der Wahrnehmungselektrode zu verarbeiten und eine jeweilige Kammerkontraktion zu erfassen und im Falle einer detektierten Kammerkontraktion ein ventrikuläres Sensingsignal auszugeben,
- wobei die ventrikuläre Wahrnehmungseinheit ausgebildet ist, die Signale der Wahrnehmungselektrode mit mindestens zwei umschaltbaren Sensingschwellen wie folgt zu verarbeiten:
- nach jeder Wahrnehmung wird zunächst ein VF-Erkennungsfenster auf einer ersten unteren Schwelle als erster Sensingschwelle gestartet und
- erst nach Ablauf des VF-Erkennungsfensters ein T-Wellenausblendungsfenster auf einer oberen Schwelle als zweiter Sensingschwelle aktiviert und
- nach Ablauf des T-Wellenausblendungsfensters dann die Wahrnehmung auf einer zweiten unteren Schwelle gestartet.

2. Implantierbarer Herzstimulator nach Anspruch 1, **dadurch gekennzeichnet, dass** die erste und die zweite untere Schwelle identisch und von der zweiten Sensingschwelle verschieden sind.

3. Implantierbarer Herzstimulator nach Anspruch 1, **dadurch gekennzeichnet, dass** die erste und die zweite untere Schwelle unterschiedlich und von der zweiten Sensingschwelle verschieden sind.

4. Implantierbarer Herzstimulator nach einem der Ansprüche 1 bis 3, **dadurch gekennzeichnet, dass** der Herzstimulator eine Steuereinheit aufweist, die ausgebildet ist, eine jeweilige Dauer des VF-Erkennungsfensters abhängig von einer jeweils gemessenen Herzfrequenz einzustellen.

5. Implantierbarer Herzstimulator nach Anspruch 4, **dadurch gekennzeichnet, dass** die Steuereinheit ausgebildet ist, im Anschluss an ein VF-Erkennunngsfenster, in dem keine Überschreitung der ersten Sensingschwelle durch ein erfasstes Signal erfolgt, ein T-Wellen-Ausblendfenster zu starten und eine jeweilige Dauer des VF-Erkennungsfensters nur dann abhängig von einer jeweils gemessenen Herzfrequenz einzustellen, wenn in einem jeweils vorangegangenen Herzzyklus ein T-Wellenausblendfenster gestartet wurde.

6. Implantierbarer Herzstimulator nach einem der Ansprüche 1 bis 5, **dadurch gekennzeichnet, dass** der implantierbare Herzstimulator ein Einkammer-, Zweikammer-, Dreikammer-ICD ist.

7. Implantierbarer Herzstimulator nach einem der Ansprüche 1 bis 6, **dadurch gekennzeichnet, dass** die Wahrnehmungseinheit derart mit einer Wahrnehmungselektrode verbunden oder zu verbinden ist, dass eine Erfassung intrakardialer elektrischer Potentiale bipolar erfolgt.

8. Implantierbarer Herzstimulator nach einem der Ansprüche 1 bis 6, **dadurch gekennzeichnet, dass** die Wahrnehmungseinheit derart mit einer Wahrnehmungselektrode verbunden oder zu verbinden ist, dass eine Erfassung intrakardialer elektrischer Potentiale unipolar erfolgt.

9. Implantierbarer Herzstimulator nach einem der Ansprüche 1 bis 8, **dadurch gekennzeichnet, dass** die Wahrnehmungseinheit derart mit einer Wahrnehmungselektrode verbunden oder zu verbinden ist, dass sie ein Fernfeld intrakardialer elektrischer Potentiale erfasst.

10. Implantierbarer Herzstimulator nach einem der Ansprüche 1 bis 9, **dadurch gekennzeichnet, dass** die Wahrnehmungseinheit derart mit mehreren Wahrnehmungselektroden verbunden oder zu verbinden ist, dass eine Erfassung intrakardialer elektrischer Potentiale über mehrere Vektoren erfolgt.
